# EUROPEAN PATENT SPECIFICATION

(11) **EP 1 446 095 B1**
(45) Date of publication and mention of the grant of the patent: **16.01.2008**
(21) Application number: 02777976.8
(22) Date of filing: 28.10.2002
(51) Int. Cl.: A61K 8/67, A61Q 19/00, A61K 31/355

(54) **SKIN PREPARATION COMPRISING A TOCOPHEROL DERIVATIVE FOR EXTERNAL APPLICATION**
HAUTPRÄPARAT ENTHALTEND EIN TOCOPHEROLDERIVAT ZUR ÄUSSERLICHEN ANWENDUNG
PREPARATION POUR USAGE EXTERNE S'APPLIQUANT SUR LA PEAU ET CONTENANT UN DERIVE DE TOCOPHEROL

(30) Priority: 29.10.2001 JP 2001331581; 26.02.2002 US 359334 P; 12.04.2002 JP 2002110107; 19.04.2002 US 373579 P
(43) Date of publication of application: 18.08.2004
(73) Proprietor: Showa Denko K.K., Tokyo 105-8518 (JP)
(72) Inventor: KATO, Eiko, c/o SHOWA DENKO K.K., Chiba-shi, Chiba 267-0056 (JP); TSUZUKI, Toshi, c/o SHOWA DENKO K.K., Chiba-shi, Chiba 267-0056 (JP); TAKATA, Jiro, Fukuoka-shi, Fukuoka 819-0044 (JP); KARUBE, Yoshiharu, Fukuoka-shi, Fukuoka 814-0144 (JP); MATSUNAGA, Kazuhisa, Fukuoka-shi, Fukuokaken 814-0133 (JP); KOBAYASHI, Shizuko, Tokyo 177-0034 (JP)
(74) Representative: Fischer, Heinrich
(86) International application number: PCT/JP2002/011152
(87) International publication number: WO 2003/037290

(56) References cited:
- US-A- 2 988 553
- PATENT ABSTRACTS OF JAPAN vol. 008, no. 085 (C-219), 18 April 1984 (1984-04-18) -& JP 59 007112 A (SHISEIDO KK), 14 January 1984 (1984-01-14)
- PATENT ABSTRACTS OF JAPAN vol. 008, no. 046 (C-212), 29 February 1984 (1984-02-29) -& JP 58 203982 A (SHISEIDO KK), 28 November 1983 (1983-11-28) cited in the application
- JIRO TAKATA ET AL: "PRODRUGS OF VITAMIN E.1" JOURNAL OF PHARMACEUTICAL SCIENCES, AMERICAN PHARMACEUTICAL ASSOCIATION. WASHINGTON, US, vol. 84, no. 1, 1995, pages 96-100, XP002092803 ISSN: 0022-3549
- PATENT ABSTRACTS OF JAPAN vol. 014, no. 397 (C-0752), 28 August 1990 (1990-08-28) -& JP 02 149576 A (EISAI CO LTD), 8 June 1990 (1990-06-08)
- PATENT ABSTRACTS OF JAPAN vol. 014, no. 397 (C-0752), 28 August 1990 (1990-08-28) -& JP 02 149577 A (EISAI CO LTD), 8 June 1990 (1990-06-08)
- PATENT ABSTRACTS OF JAPAN vol. 013, no. 362 (C-625), 14 August 1989 (1989-08-14) -& JP 01 121285 A (EISAI CO LTD), 12 May 1989 (1989-05-12)
- PATENT ABSTRACTS OF JAPAN vol. 013, no. 362 (C-625), 14 August 1989 (1989-08-14) -& JP 01 121284 A (EISAI CO LTD), 12 May 1989 (1989-05-12)
- DATABASE BIOSIS [Online] BIOSCIENCES INFORMATION SERVICE, PHILADELPHIA, PA, US; 1997 TAKATA JIRO ET AL: "Water-soluble prodrug of vitamin E for parenteral use and its effect on endotoxin induced liver toxicity." Database accession no. PREV199799511062 XP002230111 & BIOLOGICAL & PHARMACEUTICAL BULLETIN, vol. 20, no. 2, 1997, pages 204-209, ISSN: 0918-6158
- DATABASE BIOSIS [Online] BIOSCIENCES INFORMATION SERVICE, PHILADELPHIA, PA, US; July 1999 (1999-07) NAGATA YOSHIKO ET AL: "Effects of a water-soluble prodrug of vitamin E on doxorubicin-induced toxicity in mice." Database accession no. PREV199900492534 XP002230112 & BIOLOGICAL & PHARMACEUTICAL BULLETIN, vol. 22, no. 7, July 1999 (1999-07), pages 698-702, ISSN: 0918-6158

## Description

### Technical Field

The present invention relates to the cosmetic use of an aqueous skin preparation for external application in which a tocopherol aminoalkylcarboxylate ester having a substituent on the N atom and/or a salt thereof is contained.

### Background Art

Tocopherols (e.g., α-tocopherol, β-tocopherol, γ-tocopherol, δ-tocopherol) known as vitamin E and derivatives thereof such as tocopherol acetate and tocopherol nicotinate are known to provide efficacy and effect such as activities of antioxidation, vital membrane stabilization, immunoactivation and acceleration of blood circulation and have been long blended in medical preparations, cosmetics, samples and the like.

However, these compounds are oil-soluble and cannot be uniformly dispersed in an aqueous solution or an emulsion. In the case of preparing a medical or cosmetic product in the solubilized or emulsion state, a nonionic surfactant is generally used to enable uniform dispersion, however, some nonionic surfactants are highly irritating or give rise to environmental pollution and therefore, in view of safety, use of the nonionic surfactant is considered undesirable and improvement is demanded in this point.

Furthermore, tocopherols in the simple form are readily oxidized and unstable and therefore, are used as an organic acid ester derivative such as acetate ester, nicotinate ester or succinate ester in many cases. In order to allow the organic acid ester derivative to exert in vivo the physiological activity as tocopherol, the ester bond moiety must be hydrolyzed by an enzyme such as esterase, however, the conversion rate of those derivatives is not sufficiently high and the effect of increasing the concentration in the tissue is low.

JP 59-007112 and JP 58-203982 disclose non-aqueous vitamin E amino acid esters.

Takata et al., Journal of Pharmaceutical Sciences, Vol. 84, No. 1, 1995, p. 96-100, disclose aminoalkanecarboxylic acid esters of d-α-tocopherol.

JP 02-149576 and JP 02-149577 disclose a bile acid salt of a tocopherol aminoalkylcarboxylic acid ester for medical use.

JP1121285 discloses tocopherol N,N-dialkylaminoalkyl-carboxylic acid esters and hydrohalides thereof.

US 2,988,553 discloses α-tocopherol-N,N-dialkylesters.

Takata et al., DATABASE BIOSIS [Online] BIOSCIENCES INFORMATION SERVICES, PHILADELPHIA, PA, US; 1997, XP002230111 Database accesion no. PREV199799511062 and Nagata et al., DATABASE BIOSIS [Online] BIOSCIENCES INFORMATION SERVICES, PHILADELPHIA, PA, US; 1999, XP002230112 Database accesion no. PREV199900492534 disclose d-alpha-tocopheryl-N,N-dimethyl-aminoacetate hadrochloride as a prodrug.

It is an object of the present invention to improve the solubility and emulsifiability of tocopherol in skin preparations for external application and provide a composition which undergoes efficient conversion to active tocopherol in the skin tissue.

### DISCLOSURE OF THE INVENTION

As a result of extensive investigations to overcome the above-described problems, the present inventors have found that a specific tocopherol aminoalkylcarboxylate ester having a substituent on the N atom and/or a salt thereof have useful solubility and emulsifiability, and have accomplished the present invention. As used herein, "having a substituent on the N atom" means to have a substituent other than an alkylcarboxylate group on an amino group of the aminoalkylcarboxylate.

The present inventors have also found that the tocopherol aminoalkylcarboxylate ester having a substituent on the N atom and/or a salt thereof are efficiently converted to active tocopherol in skin tissue, and have accomplished the present invention.

More specifically, the present invention relates to subject-matter defined in the claims.

### BEST MODE FOR CARRYING OUT THE INVENTION

The tocopherol aminoalkylcarboxylate ester derivative having an amino group having a substituent on the N atom and/or a salt thereof used in the skin preparation for external application of the present invention are described below

In the present invention, the used tocopherol aminoalkyl-carboxylate ester having an amino group having a substituent on the N atom is a compound represented by the following formula (I) and/or a salt thereof: 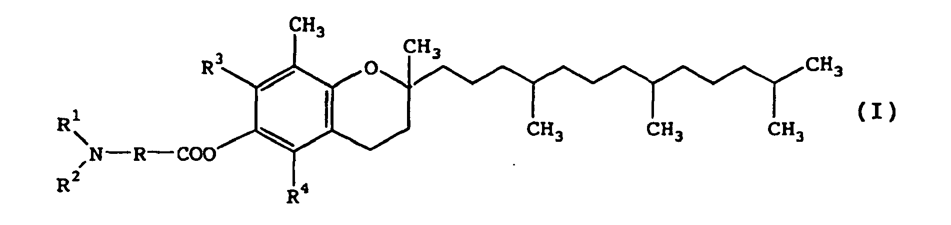 (wherein R¹ and R² may be the same or different and each represents a branched or linear alkyl group having 1 to 6 carbons atoms or a hydrogen atom, R³ and R⁴ each represents a hydrogen atom or a methyl group and R represents a branched or linear alkylene group which may have a substituent, provided that R¹ and R² are not a hydrogen atom at the same time).

As seen from the formula above, the tocopherol which is used in the present invention includes α-, β-, γ- and δ-tocopherol derivatives. Among these, preferred are α-tocopherol where R³ and R⁴ are methyl, and γ-tocopherol where R³ is methyl and R⁴ is a hydrogen atom.

These tocopherol derivatives have an asymmetric carbon atom at the 2-position of the chromanol ring and therefore, steric isomers such as d form and dl form are present. Needless to say, the present invention includes all of these isomers.

The lower alkyl group in the definition of R¹ and R² of formula (I) is a linear or branched alkyl group having from 1 to 6 carbon atoms and examples thereof include methyl, ethyl, n-propyl, n-butyl, isopropyl, isobutyl, 1-methylpropyl, tert-butyl, n-pentyl, 1-ethylpropyl, isoamyl and n-hexyl. Among these, most preferred are a methyl group and an ethyl group.

Examples of the aminoalkylcarboxylic acids constituting the tocopherol aminoalkylcarboxylate ester for use in the present invention include glycine, alanine, β-alanine, valine, leucine, isoleucine, phenylalanine, methionine, cysteine, serine, threonine, tyrosine, thyroxine, histidine, proline, 4-hydroxyproline, aspartic acid, glutamic acid and their N-alkyl derivatives and N,N-dialkyl derivatives provided that R¹ and R² are not a hydrogen atom at the same time.

Among these aminoalkylcarboxylic acids, preferred are dimethylglycine and sarcosine.

These aminoalkylcarboxylic acids may be any of D form, L form and DL form but in view of bioactivity and the like, L form or DL form is preferred.

In the present invention, a salt is preferred and the salt is preferably a hydrohalogenic acid salt, more preferably an HCl salt or an HBr salt. In particular, the HCl salt is advantageous in that the solubility in water increases and due to its powder form, handling is facilitated.

The tocopherol aminoalkylcarboxylate ester derivative having a substituent on the N atom used in the present invention may be produced by various methods, but a representative method is described below.

The production method is described by referring to the case of R=(CH₂)ₙ (wherein n represents an integer of 1 to 7) which is a preferred example.

This compound can be easily obtained by performing an esterification reaction of a tocopherol represented by the following formula (III) : 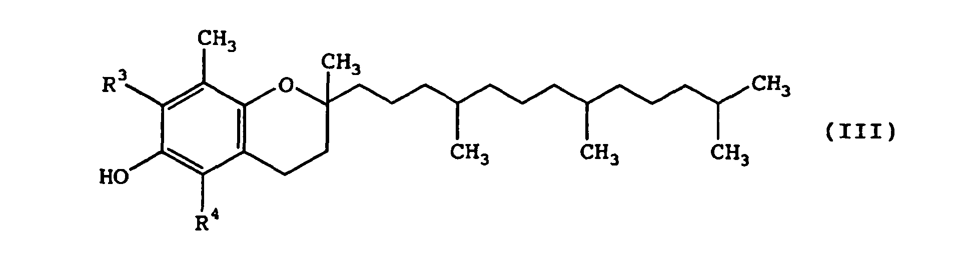 (wherein R³ and R⁴ each represents a hydrogen atom or a methyl group) and any one of an aminoalkylcarboxylic acid represented by the following formula (IV): 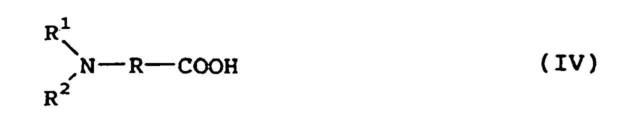 (wherein R¹ and R² may be the same or different and each represents a branched or linear alkyl group having 1 to 6 carbons atoms or a hydrogen atom, and R represents a branched or linear alkylene group which may have a substituent, provided that R¹ and R² are not a hydrogen atom at the same time), its reactive acid derivative and a salt thereof such as hydrohalogenic acid salt, in a usual manner.

In the case of directly performing the esterification using a free aminoalkylcarboxylic acid, usually, the reaction is preferably performed in the presence of an active esterification reagent (dehydrating agent) such as dicyclohexylcarbodiimide and N,N-disuccinimide oxalate. At this time, the solvent is most preferably pyridine.

If desired, the aminoalkylcarboxylic acid having a substituent on the N atom after the completion of reaction is preferably subjected to a treatment for removing the protective group using an aminoalkylcarboxylic acid in which the amino group is protected, for example, by an N-tert-butoxycarbonyl (BOC) group, a benzyloxycarbonyl group or a 2-nitrobenzenesulfonyl group.

In the method of using a reactive acid derivative, an acid halide, particularly acid chloride is preferably used.

In the case of producing a hydrohalogenic acid salt of a tocopherol aminoalkylcarboxylate ester, the hydrohalogenic acid salt may be produced by once producing an ester form and reacting it with a hydrohalogenic acid (gas phase or solution) in a usual manner, or a hydrohalogenic acid salt of an aminoalkylcarboxylic acid represented by formula (IV) may be previously used as a starting material.

The thus-obtained tocopherol aminoalkylcarboxylate ester having a substituent on the N atom and/or a hydrohalogenic acid salt thereof are excellent in the solubility and emulsifiability as compared with tocopherols in a simple form. Furthermore, when applied as a skin preparation for external application, these are readily hydrolyzed by an esterase or carboxyl esterase in the skin tissue to produce an active free tocopherol.

Therefore, the tocopherol aminoalkylcarboxylate ester having a substituent on the N atom and/or a hydrohalogenic acid salt thereof is used in the present invention as an active ingredient of skin preparations for external application which are expected to have efficacy and effect such as activities of antioxidation, vital membrane stabilization, immunoactivation and acceleration of blood circulation.

The present invention relates to the cosmetic use of an aqueous a skin preparation for external application where a tocopherol aminoalkylcarboxylate ester having a substituent on the N atom and/or a hydrohalogenic acid salt thereof is blended.

The form of the cosmetic use of the present invention includes, in a wide sense, cosmetic materials which come into contact with skin on use, for example, skin milk, skin cream, foundation cream, massage cream, cleansing cream, shaving cream, cleansing foam, skin lotion, lotion, pack, shampoo, rinse, hair restorer, hair nourishment, hair dye, hair conditioner, toothpaste, gargle, permanent waving agent, ointment, bath preparation and body soap. The user may be any user irrespective of sex or age.

In the skin preparation for external application used in the present invention, ingredients commonly used in skin preparations for external application can be blended within the range of not impairing the effect of the present invention. Examples thereof include chemicals described in Japanese Standards of Cosmetic Ingredients (JSCI), 2nd Edition, Annotation, compiled by Nippon Koteisho Kyokai, issued by Yakuji Nippo, Ltd. (1984), Specifications of Ingredient Other Than Those Listed in JSCI, supervised by Examination Division, Pharmaceutical Affairs Bureau, Ministry of Health and Welfare, issued by Yakuji Nippo, Ltd. (1993), Specifications of Ingredient Other Than Those Listed in JSCI, Supplement, supervised by Examination Division, Pharmaceutical Affairs Bureau, Ministry of Health and Welfare, issued by Yakuji Nippo, Ltd. (1993), The Comprehensive Licensing Standards of Cosmetics by Category, supervised by Examination Division, Pharmaceutical Affairs Bureau, Ministry of Health and Welfare, issued by Yakuji Nippo, Ltd. (1993), and Keshohin Genryo Jiten (Handbook of Cosmetic Ingredients), Nikko Chemicals (1991).

### Examples

The present invention is described in greater detail below by referring to Examples, however, the present invention is not limited to these Examples. In Examples, the amount blended is in the unit of mass%.

### Example 1

### Lotion 1

| | | Example 1 |
|---|---|---|
| 1) | α-Tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Example 1)

Ingredients 1) and 2) to 4) were uniformly dispersed and dissolved and the resulting solution was added to 5) with stirring to obtain the objective lotion.

### Example 2

### Lotion 2

| | | Example 2 |
|---|---|---|
| 1) | α-Tocopherol sarcosine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Example 2)

Ingredients 1) and 2) to 4) were uniformly dispersed and dissolved and the resulting solution was added to 5) with stirring to obtain the objective lotion.

### Comparative Example 1

### Lotion 3

| | | Comparative Example 1 |
|---|---|---|
| 1) | Tocopherol acetate | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Comparative Example 1)

Ingredients 1) to 4) were uniformly dispersed and dissolved and the resulting solution was added to 5) with stirring to obtain the objective lotion.

### (Results)

Lotion 1 obtained in Examples 1 and 2 was uniformly dissolved and exhibited good aging stability. On the other hand, in Comparative Example 1, uniform dissolution or dispersion could not be attained and a lotion having excellent solubility could not be obtained.

### Example 3

### Lotion 4

| | | Example 3 |
|---|---|---|
| 1) | α-Tocopherol dimethylglycine ester hydrochloride | 0.10 |
| 2) | Propylene glycol | 5.00 |
| 3) | Methyl parahydroxybenzoate | 0.20 |
| 4) | Purified water | 94.7 |

### (Production Method of Example 3)

Ingredients 1), 2) and 3) were uniformly dispersed and dissolved and the resulting solution was added to 4) with stirring to obtain the objective lotion.

### Example 4

### Lotion 5

| | | Example 4 |
|---|---|---|
| 1) | α-Tocopherol sarcosine ester hydrochloride | 0.10 |
| 2) | Propylene glycol | 5.00 |
| 3) | Methyl parahydroxybenzoate | 0.20 |
| 4) | Purified water | 94.7 |

### (Production Method of Example 4)

Ingredients 1), 2) and 3) were uniformly dispersed and dissolved and the resulting solution was added to 4) with stirring to obtain the objective lotion.

### Comparative Example 2

### Lotion 6

| | | Comparative Example 2 |
|---|---|---|
| 1) | Tocopherol acetate | 0.10 |
| 2) | Propylene glycol | 5.00 |
| 3) | Methyl parahydroxybenzoate | 0.20 |
| 4) | Purified water | 94.7 |

### (Production Method of Comparative Example 2)

Ingredients 1), 2) and 3) were uniformly dispersed and dissolved and the resulting solution was added to 4) with stirring to obtain the objective lotion.

### (Results)

Lotions 4 and 5 obtained in Examples 3 and 4 were uniformly dissolved and exhibited good aging stability. On the other hand, in Comparative Example 2, uniform dissolution or dispersion could not be attained, floating of oil droplets was confirmed and a lotion 6 having excellent solubility could not be obtained.

### Example 5

### Lotion 7

| | | Example 5 |
|---|---|---|
| 1) | α-Tocopherol dimethylglycine ester | 0.10 |
| | hydrochloride | |
| 2) | Propylene glycol | 5.00 |
| 3) | Methyl parahydroxybenzoate | 0.20 |
| 4) | Sodium ascorbyl phosphate | 3.00 |
| 5) | Purified water | 91.7 |

### (Production Method of Example 5)

Ingredients 1), 2) and 3) were uniformly dispersed and dissolved and the resulting solution was added with stirring to 5) in which 4) was previously dissolved, to obtain the objective lotion.

### Example 6

### Lotion 8

| | | Example 6 |
|---|---|---|
| 1) | α-Tocopherol sarcosine ester hydrochloride | 0.10 |
| 2) | Propylene glycol | 5.00 |
| 3) | Methyl parahydroxybenzoate | 0.20 |
| 4) | Magnesium ascorbyl phosphate | 3.00 |
| 5) | Purified water | 91.7 |

### (Production Method of Example 6)

Ingredients 1), 2) and 3) were uniformly dispersed and dissolved and the resulting solution was added with stirring to 5) in which 4) was previously dissolved, to obtain the objective lotion.

### Comparative Example 3

### Lotion 9

| | | Comparative Example 3 |
|---|---|---|
| 1) | Tocopherol acetate | 0.10 |
| 2) | Propylene glycol | 5.00 |
| 3) | Methyl parahydroxybenzoate | 0.20 |
| 4) | Sodium ascorbyl phosphate | 3.00 |
| 5) | Purified water | 91.7 |

### (Production Method of Comparative Example 3)

Ingredients 1), 2) and 3) were uniformly dispersed and dissolved and the resulting solution was added with stirring to 5) in which 4) was previously dissolved, to obtain the objective lotion.

### (Results)

Lotions 7 and 8 obtained in Examples 5 and 6 were uniformly dissolved and exhibited good aging stability. On the other hand, in Comparative Example 3, uniform dissolution or dispersion could not be attained, floating of oil droplets was confirmed and a lotion having excellent solubility could not be obtained.

### Comparative Example 7

### Gel Preparation 1 for External Application

| | | Example 7 |
|---|---|---|
| 1) | α-Tocopherol dimethylglycine ester hydrochloride | 10.0 |
| 2) | Glycerin | 20.0 |
| 3) | Octyldodecyl myristate | 70.0 |

### (Production Method of Example 7)

Ingredient 1) was uniformly dispersed in 2) and the resulting dispersion was added to 3) with stirring to obtain the objective gel preparation 1 for external application.

### Comparative Example 8

### Gel Preparation 2 for External Application

| | | Example 8 |
|---|---|---|
| 1) | α-Tocopherol sarcosine ester hydrochloride | 10.0 |
| 2) | Glycerin | 20.0 |
| 3) | Octyldodecyl myristate | 70.0 |

### (Production Method of Example 8)

Ingredient 1) was uniformly dispersed in 2) and the resulting dispersion was added to 3) with stirring to obtain the objective gel preparation 2 for external application.

### Comparative Example 4

### Gel Preparation 3 for External Application

| | | Comparative Example 4 |
|---|---|---|
| 1) | Tocopherol acetate | 10.0 |
| 2) | Glycerin | 20.0 |
| 3) | Octyldodecyl myristate | 70.0 |

### (Production Method of Comparative Example 4)

Ingredient 1) was uniformly dispersed in 2) and the resulting dispersion was added to 3) with stirring to obtain the objective gel preparation 3 for external application.

### (Results)

Gel preparations 1 and 2 for external application obtained in Examples 7 and 8 had a translucent gel appearance and exhibited good aging stability. On the other hand, in Comparative Example 4, gel was not formed.

### Example 9

Milky Lotion 1

| | | Example 9 |
|---|---|---|
| 1) | α-Tocopherol dimethylglycine ester hydrochloride | 5.0 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Example 9)

Ingredient 1) was uniformly dispersed and dissolved in 2) to 4) and this was added to 5) with stirring to obtain the objective milky lotion 1.

### Example 10

### Milky Lotion 2

| | | Example 10 |
|---|---|---|
| 1) | α-Tocopherol sarcosine ester hydrochloride | 5.0 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Example 10)

Ingredient 1) was uniformly dispersed and dissolved in 2) to 4) and this was added to 5) with stirring to obtain the objective milky lotion 2.

### Comparative Example 5

### Milky Lotion 3

| | | Comparative Example 5 |
|---|---|---|
| 1) | Tocopherol acetate | 5.00 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Comparative Example 5)

Ingredient 1) was uniformly dispersed and dissolved in 2) to 4) and this was added to 5) with stirring to obtain the objective milky lotion 3.

### (Results)

Milky lotions 1 and 2 obtained in Examples 9 and 10 gave good feeling on use and exhibited good aging stability. On the other hand, in Comparative Example 5, emulsion was not formed and a milky lotion could not be obtained.

### Example 11

### Milky Lotion 4

| | | Example 11 |
|---|---|---|
| 1) | α-Tocopherol dimethylglycine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Example 11)

Ingredient 1) was uniformly dispersed and dissolved in 2) to 4) and this was added, with stirring, to 6) in which 5) was previously dissolved to obtain the objective milky lotion 4.

### Example 12

### Milky Lotion 5

| | | Example 12 |
|---|---|---|
| 1) | α-Tocopherol dimethylglycine ester hydrochloride | 5.0 |
| 2) | Propylene glycol | 10.0 |
| 3) | Methyl parahydroxybenzoate | 0.20 |
| 4) | Methylphenylpolysiloxane | 20.0 |
| 5) | Magnesium ascorbyl phosphate | 3.00 |
| 6) | Purified water | 61.8 |

### (Production Method of Example 12)

Ingredient 1) was uniformly dispersed and dissolved in 2) to 4) and this was added, with stirring, to 6) in which 5) was previously dissolved to obtain the objective milky lotion 5.

### Example 13

### Milky Lotion 6

| | | Example 13 |
|---|---|---|
| 1) | α-Tocopherol sarcosine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Example 13)

Any one of 1) to 3) was -uniformly dispersed and dissolved in 5) to 6) and thereto, 9) in which 8) was previously dissolved was added with stirring to obtain the objective milky lotion 6.

### Example 14

### Milky Lotion 7

| | | Example 14 |
|---|---|---|
| 1) | α-Tocopherol sarcosine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Magnesium ascorbyl phosphate | 3.00 |
| 6) | Purified water | 61.8 |

### (Production Method of Example 14)

Ingredient 1) was uniformly dispersed and dissolved in 2) to 4) and this was added, with stirring, to 6) in which 5) was previously dissolved to obtain the objective milky lotion 7.

### Comparative Example 6

### Milky Lotion 8

| | | Comparative Example 6 |
|---|---|---|
| 1) | Tocopherol acetate | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Comparative Example 6)

Ingredient 1) was uniformly dispersed and dissolved in 2) to 4) and the resulting solution was added with stirring to 6) in which 5) was previously dissolved, to obtain the objective milky lotion 8.

### (Results)

Milky lotions 4 to 7 obtained in Examples 11 to 14 gave good feeling on use and exhibited good aging stability. On the other hand, in Comparative Example 6, emulsion was not formed and a milky lotion could not be obtained.

### Example 15

### Milky Lotion 9

| | | Example 15 |
|---|---|---|
| 1) | α-Tocopherol dimethylglycine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Example 15)

Ingredient 1) was uniformly dispersed and dissolved in 2) to 4) and this was added, with stirring, to 6) in which 5) was previously dissolved to obtain the objective milky lotion 9.

### Example 16

### Milky Lotion 10

| | | Example 16 |
|---|---|---|
| 1) | α-Tocopherol dimethylglycine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Magnesium ascorbyl phosphate | 2.00 |
| 6) | Purified water | 61.8 |

### (Production Method of Example 16)

Ingredient 1) was uniformly dispersed and dissolved in 2) to 4) and this was added, with stirring, to 6) in which 5) was previously dissolved to obtain the objective milky lotion 10.

### Example 17

### Milky Lotion 11

| | | Example 17 |
|---|---|---|
| 1) | α-Tocopherol sarcosine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Example 17)

Ingredient 1) was uniformly dispersed and dissolved in 2) to 4) and this was added to 6) in which 5) was previously dissolved with stirring to obtain the objective milky lotion 11.

### Example 18

### Milky Lotion 12

| | | Example 18 |
|---|---|---|
| 1) | α-Tocopherol sarcosine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Magnesium ascorbyl phosphate | 2.00 |
| 6) | Purified water | 61.8 |

### (Production Method of Example 17)

Ingredient 1) was uniformly dispersed and dissolved in 2) to 4) and this was added, with stirring, to 6) in which 5) was previously dissolved to obtain the objective milky lotion 12.

### Comparative Example 7

### Milky Lotion 13

| | | Comparative Example 7 |
|---|---|---|
| 1) | Tocopherol acetate | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Comparative Example 7)

Ingredient 1) was uniformly dispersed and dissolved in 2) to 4) and the resulting solution was added with stirring to 6) in which 5) was previously dissolved, to obtain the objective milky lotion 13.

### (Results)

Milky lotions 9 to 12 obtained in Examples 15 to 18 gave good feeling on use and exhibited good aging stability. On the other hand, in the milky lotion 13 obtained in Comparative Example 7, phase separation was observed after a few days and good aging stability could not be obtained. Example 19 and Comparative Example 8

### Evaluation of Skin Penetrability

### (Method)

In each of φ35 mm plastic Petri dishes, 1 ml of a Dulbecco's MEM medium containing 1), 2), 3) or 4) was placed and a nylon mesh and a lens paper were sequentially laid thereon. On the lens paper, a skin removed from the back of a hairless mouse was placed such that the epidermis came into contact with the lens paper. At this time, the dermis side was covered with a parafilm and thereby prevented from drying.

| | | |
|---|---|---|
| 1) | Not added | |
| 2) | α-Tocopherol dimethylglycine ester hydrochloride | 0.50 |
| 3) | α-Tocopherol sarcosine ester hydrochloride | 0.50 |
| 4) | Tocopherol acetate | 0.50 |

After the passage of 4 hours at 37°C, the skin was washed with a phosphoric acid buffer solution and homogenized. Then, the amount of α-tocopherol in the skin was measured. The determination of α-tocopherol was performed by high performance liquid chromatography.

The conditions for measurement by high performance liquid chromatography were as follows.

| | |
|---|---|
| Column: | Shodex ODSpak F-411 |
| Temperature: | 40°C |
| Eluent: | methanol/acetonitrile = 7/3 (containing 0.02M acetic acid and 0.02M sodium acetate) |
| Flow rate: | 0.7 ml |
| Detection: | fluorescent, Ex: 298 nm, Em: 325 nm |

### (Results)

1) 10 nmol/g of skin
2) 17 nmol/g of skin
3) 20 nmol/g of skin
4) 11 nmol/g of skin

The skin treated with α-tocopherol dimethylglycine ester hydrochloride or α-tocopherol sarcosine ester hydrochloride had significant increase of the α-tocopherol amount.

### Example 20 and Comparative Example 9

### Conversion to α-Tocopherol in Keratinocyte of Human Epidermis

### (Method)

Commercially available keratinocytes of normal human epidermis were cultured in the medium attached. The cells were harvested and spalled by freeze-thawing method. To this cell spall solution, 1), 2), 3) or 4) was added to have a final concentration of 1 mM. The resulting solution was kept at 37°C for 2 hours and then the amount of α-tocopherol liberated in the reaction solution was measured. The determination of α-tocopherol was performed by high performance liquid chromatography.

The conditions for measurement by high performance liquid chromatography were as follows.

| | |
|---|---|
| Column: | Shodex ODSpak F-411 |
| Temperature: | 40°C |
| Eluent: | methanol/acetonitrile = 7/3 (containing 0.02M acetic acid and 0.02M sodium acetate) |
| Flow rate: | 0.7 ml |
| Detection: | fluorescent, Ex: 298 nm, Em: 325 nm |

1) Not added
2) α-Tocopherol dimethylglycine ester hydrochloride
3) α-Tocopherol sarcosine ester hydrochloride
4) Tocopherol acetate

### (Results)

1) lower than detection limit
2) 6.9 nmol/ml of cell suspension
3) 26.9 nmol/ml of cell suspension
4) 0.5 nmol/ml of cell suspension

The cell spall solution in which α-tocopherol dimethylglycine ester hydrochloride or α-tocopherol sarcosine ester hydrochloride was added, had significant increase of the α-tocopherol amount.

### Example 21

### Lotion 10

| | | Example 21 |
|---|---|---|
| 1) | γ-Tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Example 21)

Ingredients 1) and 2) to 4) were uniformly dispersed and dissolved and the resulting solution was added to 5) with stirring to obtain the objective lotion 10.

### Example 22

### Lotion 11

| | | Example 22 |
|---|---|---|
| 1) | γ-Tocopherol sarcosine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Example 22)

Ingredients 1) and 2) to 4) were uniformly dispersed and dissolved and the resulting solution was added to 5) with stirring to obtain the objective lotion 11.

### Comparative Example 10

### Lotion 12

| | | Comparative Example 10 |
|---|---|---|
| 1) | γ-Tocopherol | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### (Production Method of Comparative Example 10)

Ingredients 1) and 2) to 4) were uniformly dispersed and dissolved and the resulting solution was added to 5) with stirring to obtain the objective lotion 12.

### (Results)

Lotions 10 and 11 obtained in Examples 21 and 22 was uniformly dissolved and exhibited good aging stability. On the other hand, in Comparative Example 10, uniform dissolution or dispersion could not be attained and a lotion having excellent solubility could not be obtained. Example 23

### Lotion 13

| | | Example 23 |
|---|---|---|
| ¹) | γ-Tocopherol dimethylglycine ester hydrochloride | 0.10 |
| 2) | Propylene glycol | 5.00 |
| 3) | Methyl parahydroxybenzoate | 0.20 |
| 4) | Purified water | 94.7 |

### (Production Method of Example 23)

Ingredients 1), 2) and 3) were uniformly dispersed and dissolved and the resulting solution was added to 4) with stirring to obtain the objective lotion 13.

### Example 24

### Lotion 14

| | | Example 24 |
|---|---|---|
| 1) | γ-Tocopherol sarcosine ester hydrochloride | 0.10 |
| 2) | Propylene glycol | 5.00 |
| 3) | Methyl parahydroxybenzoate | 0.20 |
| 4) | Purified water | 94.7 |

### (Production Method of Example 23)

Ingredients 1), 2) and 3) were uniformly dispersed and dissolved and the resulting solution was added to 4) with stirring to obtain the objective lotion 14.

### Comparative Example 11

### Lotion 15

| | | Comparative Example 11 |
|---|---|---|
| 1) | γ-Tocopherol | 0.10 |
| 2) | Propylene glycol | 5.00 |
| 3) | Methyl parahydroxybenzoate | 0.20 |
| 4) | Purified water | 94.7 |

### (Production Method of Comparative Example 11)

Ingredients 1), 2) and 3) were uniformly dispersed and dissolved and the resulting solution was added to 4) with stirring to obtain the objective lotion 15.

### (Results)

Lotions 13 and 14 obtained in Examples 23 and 24 were uniformly dissolved and exhibited good aging stability. On the other hand, in Comparative Example 11, uniform dissolution or dispersion could not be attained, floating of oil droplets was confirmed and a lotion having excellent solubility could not be obtained.

### Example 25

### Lotion 16

| | | Example 25 |
|---|---|---|
| 1) | γ-Tocopherol dimethylglycine ester hydrochloride | 0.10 |
| 2) | Propylene glycol | 5.00 |
| 3) | Methyl parahydroxybenzoate | 0.20 |
| 4) | Sodium ascorbyl phosphate | 3.00 |
| 5) | Purified water | 91.7 |

### (Production Method of Example 25)

Ingredients 1), 2) and 3) were uniformly dispersed and dissolved and the resulting solution was added with stirring to 5) in which 4) was previously dissolved, to obtain the objective lotion 16.

### Example 26

### Lotion 17

| | | Example 26 |
|---|---|---|
| 1) | γ-Tocopherol dimethylglycine ester hydrochloride | 0.10 |
| 2) | Propylene glycol | 5.00 |
| 3) | Methyl parahydroxybenzoate | 0.20 |
| 4) | Magnesium ascorbyl phosphate | 3.00 |
| 5) | Purified water | 91.7 |

### (Production Method of Example 26)

Ingredients 1), 2) and 3) were uniformly dispersed and dissolved and the resulting solution was added with stirring to 5) in which 4) was previously dissolved, to obtain the objective lotion 17.

### Example 27

### Lotion 18

| | | Example 27 |
|---|---|---|
| 1) | γ-Tocopherol sarcosine ester hydrochloride | 0.10 |
| 2) | Propylene glycol | 5.00 |
| 3) | Methyl parahydroxybenzoate | 0.20 |
| 4) | Sodium ascorbyl phosphate | 3.00 |
| 5) | Purified water | 91.7 |

### (Production Method of Example 27)

Ingredients 1), 2) and 3) were uniformly dispersed and dissolved and the resulting solution was added with stirring to 5) in which 4) was previously dissolved, to obtain the objective lotion 18.

### Example 28

### Lotion 19

| | | Example 28 |
|---|---|---|
| 1) | γ-Tocopherol sarcosine ester hydrochloride | 0.10 |
| 2) | Propylene glycol | 5.00 |
| 3) | Methyl parahydroxybenzoate | 0.20 |
| 4) | Magnesium ascorbyl phosphate | 3.00 |
| 5) | Purified water | 91.7 |

### (Production Method of Example 28)

Ingredients 1), 2) and 3) were uniformly dispersed and dissolved and the resulting solution was added with stirring to 5) in which 4) was previously dissolved, to obtain the objective lotion 19.

### Comparative Example 12

### Lotion 20

| | | Comparative Example 12 |
|---|---|---|
| 1) | γ-Tocopherol | 0.10 |
| 2) | Propylene glycol | 5.00 |
| 3) | Methyl parahydroxybenzoate | 0.20 |
| 4) | Sodium ascorbyl phosphate | 3.00 |
| 5) | Purified water | 91.7 |

### (Production Method of Comparative Example 12)

Ingredients 1), 2) and 3) were uniformly dispersed and dissolved and the resulting solution was added with stirring to 5) in which 4) was previously dissolved, to obtain the objective lotion 20.

### (Results)

Lotions 16 to 19 obtained in Examples 25 to 28 were uniformly dissolved and exhibited good aging stability. On the other hand, in Comparative Example 12, uniform dissolution or dispersion could not be attained, floating of oil droplets was confirmed-and a lotion having excellent solubility could not be obtained.

### Comparative Example 29

### Gel Preparation 4 for External Application

| | | Example 29 |
|---|---|---|
| 1) | γ-Tocopherol dimethylglycine ester hydrochloride | 10.0 |
| 2) | Glycerin | 20.0 |
| 3) | Octyldodecyl myristate | 70.0 |

### (Production Method of Example 28)

Ingredient 1) was uniformly dispersed in 2) and the resulting dispersion was added to 3) with stirring to obtain the objective gel preparation 4 for external application.

### Comparative Example 30

### Gel Preparation 5 for External Application

| | | Example 30 |
|---|---|---|
| 1) | γ-Tocopherol sarcosine ester hydrochloride | 10.0 |
| 2) | Glycerin | 20.0 |
| 3) | Octyldodecyl myristate | 70.0 |

### (Production Method of Example 30)

Ingredient 1) was uniformly dispersed in 2) and the resulting dispersion was added to 3) with stirring to obtain the objective gel preparation 5 for external application.

### Comparative Example 13

### Gel Preparation 6 for External Application

| | | Comparative Example 13 |
|---|---|---|
| 1) | γ-Tocopherol | 10.0 |
| 2) | Glycerin | 20.0 |
| 3) | Octyldodecyl myristate | 70.0 |

### (Production Method of Comparative Example 13)

Ingredient 1) was uniformly dispersed in 2) and the resulting dispersion was added to 3) with stirring to obtain the objective gel preparation for external application.

### (Results)

Gel preparations 4 and 5 for external application obtained in Examples 29 and 30 had a translucent gel appearance and exhibited good aging stability. On the other hand, in Comparative Example 13, gel was not formed.

### Example 31

### Milky Lotion 14

| | | Example 31 |
|---|---|---|
| 1) | γ-Tocopherol dimethylglycine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Example 31)

Ingredient 1) was uniformly dispersed and dissolved in 2) to 4) and this was added to 5) with stirring to obtain the objective milky lotion 14.

### Example 32

### Milky Lotion 15

| | | Example 32 |
|---|---|---|
| 1) | γ-Tocopherol sarcosine ester hydrochloride | 5.0 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Example 32)

Ingredient 1) was uniformly dispersed and dissolved in 2) to 4) and this was added to 5) was added with stirring to obtain the objective milky lotion 15.

### Comparative Example 14

### Milky Lotion 16

| | | Comparative Example 14 |
|---|---|---|
| 1) | γ-Tocopherol | 5.00 |
| 2) | Propylene glycol | 10.0 |
| 3) | Methyl parahydroxybenzoate | 0.20 |
| 4) | Methylphenylpolysiloxane | 20.0 |
| 5) | Purified water | 64.8 |

### (Production Method of Comparative Example 14)

Ingredient 1) was uniformly dispersed and dissolved in 2) to 4) and this was added to 5) was added with stirring to obtain the objective milky lotion 16.

### (Results)

Milky lotions 14 and 15 obtained in Examples 31 and 32 gave good feeling on use and exhibited good aging stability. On the other hand, in Comparative Example 14, emulsion was not formed and a milky lotion could not be obtained.

### Example 33

### Milky Lotion 17

| | | Example 33 |
|---|---|---|
| 1) | γ-Tocopherol dimethylglycine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Example 33)

Ingredient 1) was uniformly dispersed and dissolved in 2) to 4) and this was added, with stirring, to 6) in which 5) was previously dissolved to obtain the objective milky lotion 17.

### Example 34

### Milky Lotion 18

| | | Example 34 |
|---|---|---|
| 1) | γ-Tocopherol dimethylglycine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Magnesium ascorbyl phosphate | 3.00 |
| 6) | Purified water | 61.8 |

### (Production Method of Example 34)

Ingredient 1) was uniformly dispersed and dissolved in 2) to 4) and this was added, with stirring, to 6) in which 5) was previously dissolved to obtain the objective milky lotion 18.

### Example 35

### Milky Lotion 19

| | | Example 35 |
|---|---|---|
| 1) | γ-Tocopherol sarcosine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Example 35)

Ingredient 1) was uniformly dispersed and dissolved in 2) to 4) and this was added, with stirring, to 6) in which 5) was previously dissolved to obtain the objective milky lotion 19.

### Example 36

### Milky Lotion 20

| | | Example 36 |
|---|---|---|
| 1) | γ-Tocopherol sarcosine ester hydrochloride | 5.00 |
| 2) | Propylene glycol | 10.0 |
| 3) | Methyl parahydroxybenzoate | 0.20 |
| 4) | Methylphenylpolysiloxane | 20.0 |
| 5) | Magnesium ascorbyl phosphate | 3.00 |
| 6) | Purified water | 61.8 |

### (Production Method of Example 36)

Ingredient 1) was uniformly dispersed and dissolved in 2) to 4) and this was added, with stirring, to 6) in which 5) was previously dissolved to obtain the objective milky lotion 20.

### Comparative Example 15

### Milky Lotion 21

| | | Comparative Example 15 |
|---|---|---|
| 1) | γ-Tocopherol | 5.00 |
| 2) | Propylene glycol | 10.0 |
| 3) | Methyl parahydroxybenzoate | 0.20 |
| 4) | Methylphenylpolysiloxane | 20.0 |
| 5) | Sodium ascorbyl phosphate | 3.00 |
| 6) | Purified water | 61.8 |

### (Production Method of Comparative Example 15)

Ingredient 1) was uniformly dispersed and dissolved in 2) to 4) and this was added, with stirring, to 6) in which 5) was previously dissolved to obtain the objective milky lotion 21.

### (Results)

Milky lotions 17 to 20 obtained in Examples 33 to 36 gave good feeling on use and exhibited good aging stability. On the other hand, in Comparative Example 15, emulsion was not formed and a milky lotion could not be obtained.

### Example 37

### Milky Lotion 22

| | | Example 37 |
|---|---|---|
| 1) | γ-Tocopherol dimethylglycine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Example 37)

Ingredient 1) was uniformly dispersed and dissolved in 2) to 4) and the resulting solution was added with stirring to 6) in which 5) was previously dissolved, to obtain the objective milky lotion 22.

### Example 38

### Milky Lotion 23

| | | Example 38 |
|---|---|---|
| 1) | γ-Tocopherol dimethylglycine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Magnesium ascorbyl phosphate | 2.00 |
| 6) | Purified water | 61.8 |

### (Production Method of Example 38)

Ingredient 1) was uniformly dispersed and dissolved in 2) to 4) and the resulting solution was added with stirring to 6) in which 5) was previously dissolved, to obtain the objective milky lotion 23.

### Example 39

### Milky Lotion 24

| | | Example 39 |
|---|---|---|
| 1) | γ-Tocopherol sarcosine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Example 39)

Ingredient 1) was uniformly dispersed and dissolved in 2) to 4) and the resulting solution was added with stirring to 6) in which 5) was previously dissolved, to obtain the objective milky lotion 24.

### Example 40

### Milky Lotion 25

| | | Example 40 |
|---|---|---|
| 1) | γ-Tocopherol sarcosine ester hydrochloride | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Magnesium ascorbyl phosphate | 2.00 |
| 6) | Purified water | 61.8 |

### (Production Method of Example 40)

Ingredient 1) was uniformly dispersed and dissolved in 2) to 4) and the resulting solution was added with stirring to 6) in which 5) was previously dissolved, to obtain the objective milky lotion 25.

### Comparative Example 16

### Milky Lotion 26

| | | Comparative Example 16 |
|---|---|---|
| 1) | γ-Tocopherol | 5.00 |
| 2) | Hydrogenated soybean phospholipid | 10.0 |
| 3) | Methyl parahydroxybenzoate | 0.20 |
| 4) | 2-Ethylhexanoic acid triglyceride | 20.0 |
| 5) | Sodium ascorbyl phosphate | 2.00 |
| 6) | Purified water | 61.8 |

### (Production Method of Comparative Example 16)

Ingredient 1) was uniformly dispersed and dissolved in 2) to 4) and the resulting solution was added with stirring to 6) in which 5) was previously dissolved, to obtain the milky lotion 26.

### (Results)

Milky lotions 22 to 25 obtained in Examples 37 to 40 gave good feeling on use and exhibited good aging stability. On the other hand, in the milky lotion obtained in Comparative Example 16, phase separation was observed after a few days and good aging stability could not be obtained. Example 41 and Comparative Example 17 Evaluation of Skin Penetrability

### (Method)

In each of φ35 mm plastic Petri dishes, 1 ml of a Dulbecco's MEM medium containing 1), 2) or 3) was placed and a nylon mesh and a lens paper were sequentially laid thereon. On the lens paper, a skin removed from the back of a hairless mouse was placed such that the epidermis came into contact with the lens paper. At this time, the dermis side was covered with a parafilm and thereby prevented from drying.

| | | |
|---|---|---|
| 1) | Not added | |
| 2) | γ-Tocopherol dimethylglycine ester hydrochloride | 0.50 |
| 3) | γ-Tocopherol sarcosine ester hydrochloride | 0.50 |

After the passage of 4 hours at 37°C, the skin was washed with a phosphoric acid buffer solution and homogenized. Then, the amount of γ-tocopherol in the skin was measured. The determination of γ-tocopherol was performed by high performance liquid chromatography.

The conditions for measurement by high performance liquid chromatography were as follows.

| | |
|---|---|
| Column: | Shodex ODSpak F-411 |
| Temperature: | 40°C |
| Eluent: | methanol/acetonitrile = 7/3 (containing 0.02M acetic acid and 0.02M sodium acetate) |
| Flow rate: | 0.7 ml |
| Detection: | fluorescent, Ex: 298 nm, Em: 325 nm |

### (Results)

1) lower than detection limit
2) 20 nmol/g of skin
3) 21 nmol/g of skin

The skin treated with γ-tocopherol dimethylglycine ester hydrochloride or γ-tocopherol sarcosine ester hydrochloride had significant increase of the γ-tocopherol amount.

### Example 42 and Comparative Example 18

### Conversion to γ-Tocopherol in Keratinocyte of Human Epidermis (Method)

Commercially available keratinocytes of normal human epidermis were cultured in the medium attached. The cells were harvested and spalled by freeze-thawing method. To this cell spall solution, 1), 2) or 3) was added to have a final concentration of 1 mM. The resulting solution was kept at 37°C for 2 hours and then the amount of γ-tocopherol liberated in the reaction solution was measured. The determination of γ-tocopherol was performed by high performance liquid chromatography.

The conditions for measurement by high performance liquid chromatography were as follows.

| | |
|---|---|
| Column: | Shodex ODSpak F-411 |
| Temperature: | 40°C |
| Eluent: | methanol/acetonitrile = 7/3 (containing 0.02M acetic acid and 0.02M sodium acetate) |
| Flow rate: | 0.7 ml |
| Detection: | fluorescent, Ex: 298 nm, Em: 325 nm |

1) Not added
2) γ-Tocopherol dimethylglycine ester hydrochloride
3) γ-Tocopherol sarcosine ester hydrochloride

### (Results)

1) lower than detection limit
2) 28.1 nmol/ml of cell suspension
3) 30.3 nmol/ml of cell suspension

The cell spall solution in which γ-tocopherol dimethylglycine ester hydrochloride or γ-tocopherol sarcosine ester hydrochloride was added, had significant increase of the γ-tocopherol amount.

### Permeability of three dimensional model of human skin tissue and tocopherol conversion

40 µL of a 1 % solution of the below test substances 1) to 7) dissolved or dispersed in Dulbecco's PBS (-) were applied onto the tissue surface of a three dimensional model of human skin tissue (TESTSKIN^{™} LSD-d, Toyobo K. K.) and were cultured at 37°C under 5% CO₂ for 6 hours. After this, the solutions of the test substances were removed by aspiration and sampling was carried out.

The sample model skin was washed with Dulbecco's PBS (-) and the tissue surfaces onto which the test substances were applied were punched out with a φ 6 mm punch, and were homogenated in a HEPES buffer solution (pH 7.2), and quantitative analysis of the α-tocopherol and γ-tocopherol were carried out by high speed liquid chromatography. Quantitative analysis of the amount of protein in the model skin was carried out according to the Lowry method.
1) α-tocopherol dimethylglycine ester hydrochloride
2) α-tocopherol sarcosine ester hydrochloride
3) α-tocopherol glycine ester
4) γ-tocopherol dimethylglycine ester hydrochloride
5) γ-tocopherol sarcosine ester hydrochloride
6) γ-tocopherol glycine ester
7) tocopherol acetate

The high speed liquid chromatography measurement conditions were as described below.

| | |
|---|---|
| Column: | Shodex ODSpak F-411 |
| Temperature: | 40ºC |
| Eluant: | methanol/acetonitrile = 7/3 (including 0.02 M acetic acid, 0.02 M sodium acetate) |
| Flow rate: | 0.7 ml |
| Detection: | fluorescence, Ex 298 mm, Em 325 mm |

The amounts of α-tocopherol and γ-tocopherol for the samples processed with each test substance were as follows.
1) 2.8 nmol/mg protein (the amount of α-tocopherol)
2) 3.9 nmol/mg protein (the amount of α-tocopherol)
3) 1.2 nmol/mg protein (the amount of α-tocopherol)
4) 4.7 nmol/mg protein (the amount of γ-tocopherol)
5) 5.5 nmol/mg protein (the amount of γ-tocopherol)
6) 1.5 nmol/mg protein (the amount of γ-tocopherol)
7) 1.0 nmol/mg protein (the amount of α-tocopherol)

For the processes carried out using α-tocopherol dimethylglycine ester hydrochloride, α-tocopherol sarcosine ester hydrochloride, γ-tocopherol dimethylglycine ester hydrochloride, and γ-tocopherol sarcosine ester hydrochloride, the level of tocopherol was significantly increased.

### INDUSTRIAL APPLICABILITY OF THE INVENTION

The skin preparation for external application used in the present invention comprising a specific tocopherol aminoalkylcarboxylate ester having a substituent on the N atom and/or a salt thereof is favored with improved solubility and emulsifiability of tocopherol and efficient conversion to active tocopherol in skin tissue and therefore, can be applied over a wide range such as skin preparations for external application and cosmetic materials.

## Claims

1. Cosmetic use of an aqueous skin preparation comprising a tocopherol aminoalkylcarboxylate ester having an amino group having a substituent on the N atom represented by formula (I), and/or a salt thereof: 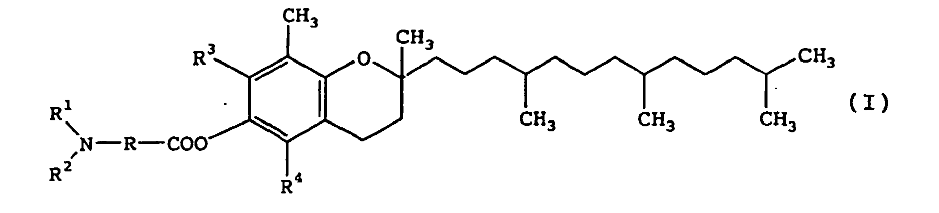 (wherein R¹ and R² may be the same or different and each represents a branched or linear alkyl group having 1 to 6 carbon atoms or a hydrogen atom, R³ and R⁴ each represents a hydrogen atom or a methyl group and R represents a branched or linear alkylene group which may have a substituent, provided that R¹ and R² are not a hydrogen atom at the same time).

2. Use according to claim 1, wherein the tocopherol aminoalkylcarboxylate ester is one or more compound selected from α-tocopherol derivatives, β-tocopherol derivatives, γ-tocopherol derivatives and δ-tocopherol derivatives.

3. Use according to claim 2, wherein the tocopherol aminoalkylcarboxylate ester is an α-tocopherol aminoalkyl-carboxylate ester or a γ-tocopherol aminoalkylcarboxylate ester.

4. Use according to claim 1, wherein the tocopherol aminoalkylcarboxylate ester having a substituent on the N atom comprises a compound represented by formula (II): 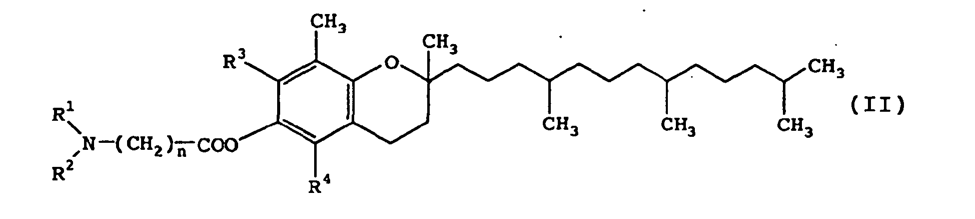 (wherein R¹ and R² may be the same or different and each represents a branched or linear alkyl group having 1 to 6 carbons atoms a hydrogen atom, R³ and R⁴ each represents a hydrogen atom or a methyl group, and n represents an integer of 1 to 7, provided that R¹ and R² are not a hydrogen atom at the same time).

5. Use according to claim 1, wherein the aminoalkylcarboxylic acid of the tocopherol aminoalkylcarboxylate ester having a substituent on the N atom is a compound selected from the group consisting of glycine, alanine, β-alanine, valine, leucine, isoleucine, phenylalanine, methionine, cysteine, serine, threonine, tyrosine, thyroxine, histidine, proline, 4-hydroxyproline, aspartic acid, glutamic acid and their N-alkyl derivatives and N,N-dialkyl derivatives.

6. Use according to claim 1, wherein the aminoalkylcarboxylic acid of the tocopherol aminoalkylcarboxylate ester having a substituent on the N atom has a monoamino group and the monoamino group is a monoalkylamino group.

7. Use according to claim 1, wherein the aminoalkylcarboxylic acid of the tocopherol aminoalkylcarboxylate ester having a substituent on the N atom has a monoamino group and the monoamino group is a dialkylamino group.

8. Use according to claim 6, wherein the tocopherol aminoalkylcarboxylate ester having a substituent on the N atom is an N,N-dimethylglycine ester of tocopherol.

9. Use according to claim 7, wherein the tocopherol aminoalkylcarboxylate ester having a substituent on the N atom is a tocopherol sarcosine ester.

10. Use according to claim 1, wherein the salt is a hydrohalogenic acid salt.

11. Use according to claim 10, wherein the hydrohalogenic acid is hydrochloric acid.

12. Use according to any one of the claims 1 to 11, wherein the content of the tocopherol aminoalkylcarboxylate ester having a substituent on the N atom and/or a salt thereof is from 0.01 to 10 mass%.

## Patentansprüche

1. Kosmetische Verwendung einer wässerigen Zubereitung für die Haut, die einen Tocopherolaminoalkylcarboxylatester mit einer Aminogruppe mit einem Substituenten am N-Atom der Formel (I) und/oder ein Salz davon enthält: 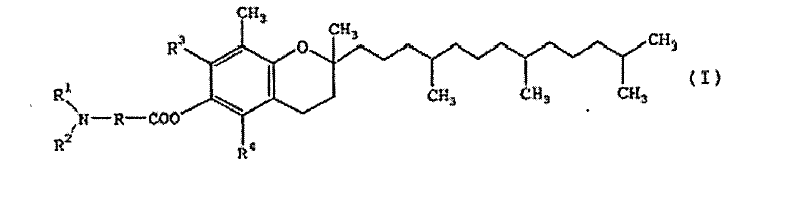 (worin R¹ und R² gleich oder unterschiedlich voneinander sein können und jeweils eine verzweigte oder geradkettige Alkylgruppe mit 1 bis 6 Kohlenstoffatomen oder ein Wasserstoffatom darstellen, R³ und R⁴ jeweils ein Wasserstoffatom oder eine Methylgruppe darstellen und R eine verzweigte oder geradkettige Alkylgruppe, die einen Substituenten aufweisen kann, darstellt, mit der Maßgabe, daß R¹ und R² nicht gleichzeitig ein Wasserstoffatom darstellen).

2. Verwendung nach Anspruch 1, wobei der Tocopherolaminoalkylcarboxylatester eine oder mehrere Verbindungen darstellt, die unter α-Tocopherolderivaten, β-Tocopherolderivaten, γ-Tocopherolderivaten und δ-Tocopherolderivaten ausgewählt sind.

3. Verwendung nach Anspruch 2, wobei der Tocopherolaminoalkylcarboxylatester ein α-Tocopherolaminoalkylcarboxylatester oder ein γ-Tocopherolaminoalkylcarboxylatester ist.

4. Verwendung nach Anspruch 1, wobei der Tocopherolaminoalkylcarboxylatester mit einem Substituenten am N-Atom eine Verbindung der Formel (II) umfaßt: 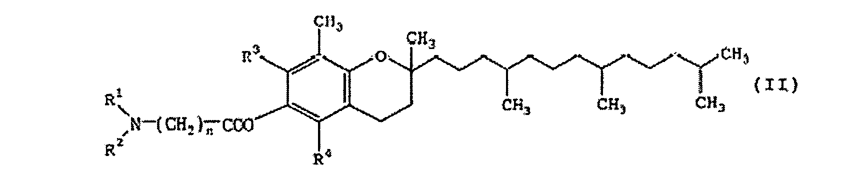 (worin R¹ und R² gleich oder unterschiedlich voneinander sein können und jeweils eine verzweigte oder geradkettige Alkylgruppe mit 1 bis 6 Kohlenstoffatomen oder ein Wasserstoffatom darstellen, R³ und R⁴ jeweils ein Wasserstoffatom oder eine Methylgruppe darstellen und n eine ganze Zahl von 1 bis 7 darstellt, mit der Maßgabe, daß R¹ und R² nicht gleichzeitig ein Wasserstoffatom sind).

5. Verwendung nach Anspruch 1, wobei Aminoalkylcarbonsäure des Tocopherolaminoalkylcarboxylatesters mit einem Substituenten am N-Atom eine Verbindung ist, die aus der Gruppe ausgewählt ist, die aus Glycin, Alanin, β-Alanin, Valin, Leucin, Isoleucin, Phenylalanin, Methionin, Cystein, Serin, Threonin, Tyrosin, Thyroxin, Histidin, Prolin, 4-Hydroxyprolin, Asparaginsäure, Glutaminsäure und deren N-Alkylderivaten und N,N-Dialkylderivaten besteht.

6. Verwendung nach Anspruch 1, wobei die Aminoalkylcarbonsäure des Tocopherolaminoalkylcarboxylatesters mit einem Substituenten am N-Atom eine Monoaminogruppe aufweist, die eine Monoalkylaminogruppe ist.

7. Verwendung nach Anspruch 1, wobei die Aminoalkylcarbonsäure des Tocopherolaminoalkylcarboxylatesters mit einem Substituenten am N-Atom eine Monoaminogruppe aufweist, die eine Dialkylaminogruppe ist.

8. Verwendung nach Anspruch 6, wobei der Tocopherolaminoalkylcarboxylatester mit einem Substituenten am N-Atom ein N,N-Dimethylglycinester von Tocopherol ist.

9. Verwendung nach Anspruch 7, wobei der Tocopherolaminoalkylcarboxylatester mit einem Substituenten am N-Atom ein Tocopherolsarcosinester ist.

10. Verwendung nach Anspruch 1, wobei das Salz ein Halogenwasserstoffsäuresalz ist.

11. Verwendung nach Anspruch 10, wobei die Halogenwaserstoffsäure Chlorwasserstoffsäure ist.

12. Verwendung nach einem der Ansprüche 1 bis 11, wobei der Gehalt des Tocopherolaminoalkylcarboxylatesters mit einem Substituenten am N-Atom und/oder des Salzes davon von 0,01 bis 10 Massen-% ist.

## Revendications

1. Utilisation cosmétique d'une préparation aqueuse pour la peau comprenant un ester de tocophérol aminoalkylcarboxylate comportant un groupe amino ayant un substituant sur l'atome N représenté par la formule (I), et/ou un sel de celui-ci : 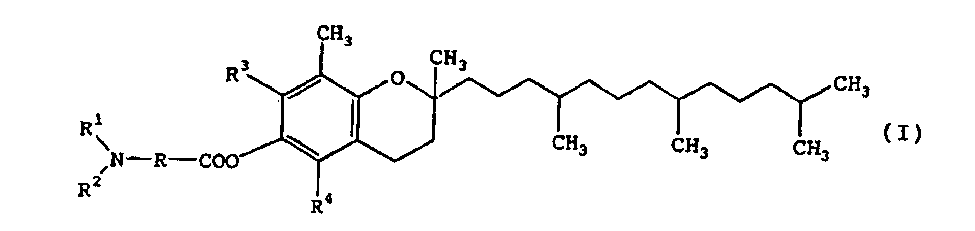 (où R¹ et R² peuvent être identiques ou différents et chacun représente un groupe alkyle ramifié ou linéaire comportant 1 à 6 atomes de carbone ou un atome d'hydrogène, R³ et R⁴ représentent chacun un atome d'hydrogène ou un groupe méthyle et R représente un groupe alkylène ramifié ou linéaire qui peut avoir un substituant, à condition que R¹ et R₂ ne soient pas un atome d'hydrogène en même temps).

2. Utilisation selon la revendication 1, dans laquelle l'ester de tocophérol aminoalkylcarboxylate est un ou plusieurs composés choisis parmi les dérivés d'α-tocophérol, les dérivés de β-tocophérol, les dérivés de γ-tocophérol et les dérivés de δ-tocophérol.

3. Utilisation selon la revendication 2, dans laquelle l'ester de tocophérol aminoalkylcarboxylate est un ester d'α-tocophérol aminoalkylcarboxylate ou un ester de γ-tocophérol aminoalkylcarboxylate.

4. Utilisation selon la revendication 1, dans laquelle l'ester de tocophérol aminoalkylcarboxylate ayant un substituant sur l'atome N comprend un composé représenté par la formule (II) : 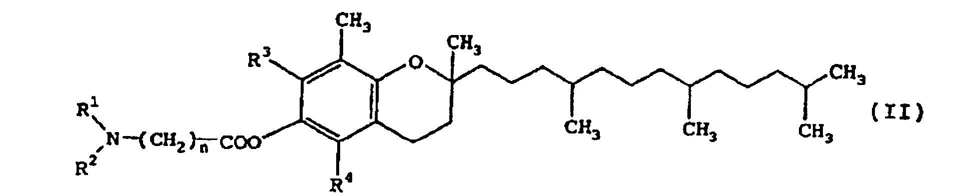 (où R¹ et R² peuvent être identiques ou différents et chacun représente un groupe alkyle ramifié ou linéaire comportant 1 à 6 atomes de carbone ou un atome d'hydrogène, R³ et R⁴ représentent chacun un atome d'hydrogène ou un groupe méthyle, et n représente un nombre entier de 1 à 7, à condition que R¹ et R₂ ne soient pas un atome d'hydrogène en même temps).

5. Utilisation selon la revendication 1, dans laquelle l'acide aminoalkylcarboxylique de l'ester de tocophérol aminoalkylcarboxylate comportant un substituant sur l'atome N est un composé choisi dans le groupe constitué par la glycine, l'alanine, la β-alanine, la valine, la leucine, l'isoleucine, la phénylalanine, la méthionine, la cystéine, la sérine, la thréonine, la tyrosine, la thyroxine, l'histidine, la proline, la 4-hydroxyproline, l'acide aspartique, l'acide glutamique et leurs dérivés N-alkyle et dérivés N,N-dialkyle.

6. Utilisation selon la revendication 1, dans laquelle l'acide aminoalkylcarboxylique de l'ester de tocophérol aminoalkylcarboxylate ayant un substituant sur l'atome N comporte un groupe monoamino et le groupe monoamino est un groupe monoalkylamino.

7. Utilisation selon la revendication 1, dans laquelle l'acide aminoalkylcarboxylique de l'ester de tocophérol aminoalkylcarboxylate ayant un substituant sur l'atome N comporte un groupe monoamino et le groupe monoamino est un groupe dialkylamino.

8. Utilisation selon la revendication 6, dans laquelle l'ester de tocophérol aminoalkylcarboxylate ayant un substituant sur l'atome N est un ester de N,N-diméthylglycine de tocophérol.

9. Utilisation selon la revendication 7, dans laquelle l'ester de tocophérol aminoalkylcarboxylate ayant un substituant sur l'atome N est un ester de tocophérol sarcosine.

10. Utilisation selon la revendication 1, dans laquelle le sel est un sel d'acide hydrohalogénique.

11. Utilisation selon la revendication 10, dans laquelle l'acide hydrohalogénique est l'acide chlorhydrique.

12. Utilisation selon l'une quelconque des revendications 1 à 11, dans laquelle la teneur en ester de tocophérol aminoalkylcarboxylate ayant un substituant sur l'atome N et/ou un sel de celui-ci est de 0,01 à 10 % en masse.
